(19) Europäisches Patentamt
European Patent Office
Office européen des brevets

(11) EP 4 620 993 A1

(12) **EUROPEAN PATENT APPLICATION**
published in accordance with Art. 153(4) EPC

(43) Date of publication:
**24.09.2025 Bulletin 2025/39**

(21) Application number: **22965507.1**

(22) Date of filing: **17.11.2022**

(51) International Patent Classification (IPC):
***C08G 18/71*** (2006.01) ***C08G 18/75*** (2006.01)
***C08G 18/73*** (2006.01) ***C08G 18/77*** (2006.01)
***C08G 18/78*** (2006.01) ***C08G 18/79*** (2006.01)
***C07C 263/04*** (2006.01) ***C07C 263/10*** (2006.01)
***C07C 265/14*** (2006.01) ***C09D 175/04*** (2006.01)

(52) Cooperative Patent Classification (CPC):
**C07C 263/04; C07C 263/10; C07C 265/14; C08G 18/71; C08G 18/73; C08G 18/75; C08G 18/77; C08G 18/78; C08G 18/79; C09D 175/04**

(86) International application number:
**PCT/CN2022/132423**

(87) International publication number:
**WO 2024/103323 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Wanhua Chemical Group Co., Ltd.**
**Yantai, Shandong 264006 (CN)**

(72) Inventors:
- **ZHU, Fulin**
**Chongqing Street, YEDA, Yantai, Shandong 264006 (CN)**
- **SHANG, Yonghua**
**Chongqing Street, YEDA, Yantai, Shandong 264006 (CN)**
- **LI, Wenbin**
**Chongqing Street, YEDA, Yantai, Shandong 264006 (CN)**
- **LI, Jianfeng**
**Chongqing Street, YEDA, Yantai, Shandong 264006 (CN)**
- **YU, Tao**
**Chongqing Street, YEDA, Yantai, Shandong 264006 (CN)**
- **WANG, Jingxu**
**Chongqing Street, YEDA, Yantai, Shandong 264006 (CN)**
- **HE, Wei**
**Chongqing Street, YEDA, Yantai, Shandong 264006 (CN)**
- **HAN, Jinping**
**Chongqing Street, YEDA, Yantai, Shandong 264006 (CN)**
- **YU, Yong**
**Chongqing Street, YEDA, Yantai, Shandong 264006 (CN)**
- **LI, Yuan**
**Chongqing Street, YEDA, Yantai, Shandong 264006 (CN)**

(74) Representative: **Murgitroyd & Company**
**165-169 Scotland Street**
**Glasgow G5 8PL (GB)**

(54) **ISOCYANATE COMPOSITION, AND PREPARATION METHOD THEREFOR AND USE THEREOF**

(57) Disclosed herein are an isocyanate composition, and a preparation method therefor and the use thereof. The effective factor of the isocyanate composition is 3.70-4.70. The isocyanate composition has good reaction activity and can be used for preparing a high-performance polyurethane product.

EP 4 620 993 A1

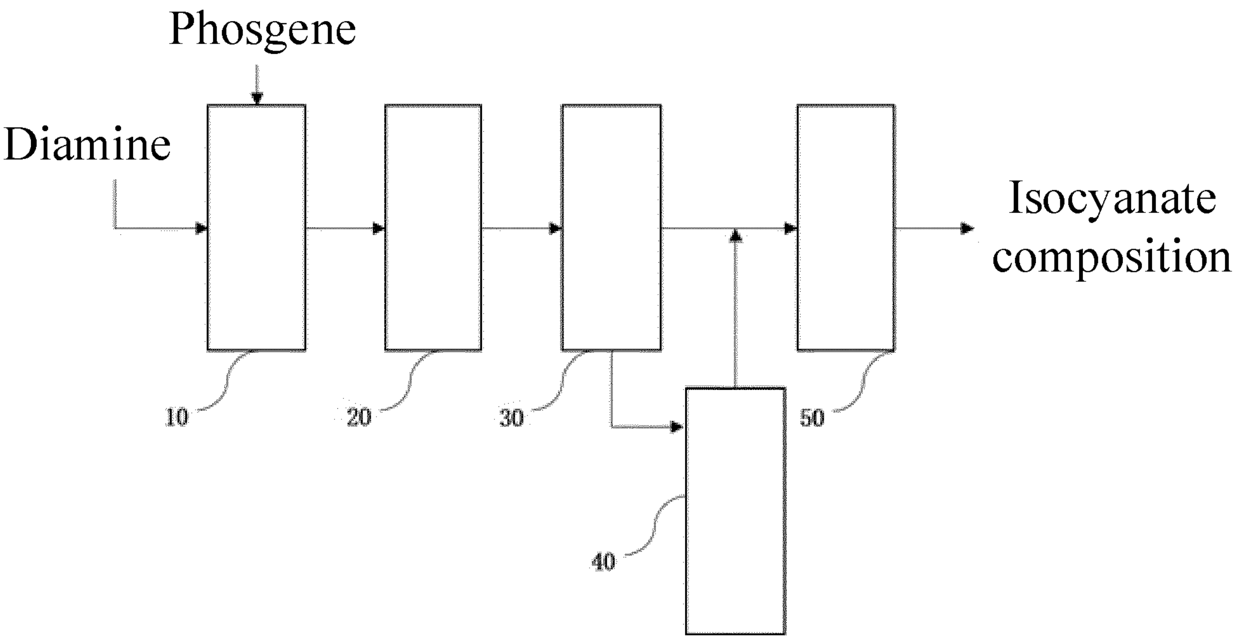


FIG. 1

## Description

TECHNICAL FIELD

Embodiments of the present application relate to the technical field of isocyanates, particularly to an isocyanate composition, a preparation method and use thereof.

BACKGROUND

Polyurethane coatings exhibit excellent performances such as low film-forming temperature, strong adhesion, good wear resistance, high hardness, good chemical resistance, good weather resistance. They are widely used in industrial protective paint, wood furniture paint, automobile original paint and automobile refinishing paint. The polyurethane coatings are typically made from isocyanates and polyols. Compared with aromatic isocyanates, aliphatic isocyanates exhibit more superior stability and are more commonly used as raw materials in polyurethane coatings. Aliphatic isocyanates can be classified into linear aliphatic isocyanates and cyclic aliphatic isocyanates depending on molecular structures. Typical examples of the former include hexamethylene diisocyanate (HDI) and the like. The latter is also referred to as cycloaliphatic isocyanates, and typical examples include dicyclohexylmethane diisocyanate (HMDI), isophorone diisocyanate (IPDI) and the like.

Although aliphatic isocyanates such as HDI, HMDI, and IPDI possess various advantages, they also have the following disadvantages: the isocyanates show a dark color due to the presence of trace impurities in the synthesis process, and the prepared polyurethane coatings and the like have insufficient discoloration resistance due to side reactions occurring in the subsequent modification processes.

The isocyanates can be prepared by reacting the corresponding amines with phosgene (carbonyl chloride). In order to remedy the disadvantages of isocyanates, researchers have focused on the preparation of isocyanates, aiming to improve the performance of the products by controlling raw materials. For example, CN101440046A discloses a method for preparing a light-colored isocyanate, which involves reacting a corresponding amine with phosgene in the presence or absence of an inert medium, wherein the amine stream fed for the phosgenation reaction has an average PRI value of less than 60 moles per million moles (mpm), the PRI refers to polarographic reducible impurities. In the preparation method, HDI is prepared by controlling the polarographic PRI value of hexamethylene diamine, and followed by polymerization reaction to obtain an HDI trimer with a low color number. CN103319372A discloses a method for preparing light-colored or colorless dicyclohexylmethane diisocyanate, which includes: a) purifying a raw material of dicyclohexylmethane diamine to obtain dicyclohexylmethane diamine containing 0.2 wt% or less of an alcohol compound, b) phosgenating the dicyclohexylmethane diamine containing 0.2 wt% or less of the alcohol compound to obtain dicyclohexylmethane diisocyanate, wherein the dicyclohexylmethane diisocyanate obtained by the method exhibits the characteristics of light color or colorless. CN1356980A discloses a light-colored isocyanate, a preparation method and use thereof, in which the isocyanate is prepared by reacting phosgene as a raw material with an amine, the phosgene containing bromine or iodine in molecular or compound form or a mixture thereof in an amount of 50 ppm or less. This method realizes the preparation of light-colored isocyanate by controlling the contents of bromide and iodide in phosgene, allowing the isocyanate to have a relatively low iodine color number IFZ. CN109761855A discloses a method for preparing isophorone diisocyanate, which includes the following steps: reacting isophorone with hydrogen cyanide to obtain isophorone nitrile; reacting isophorone nitrile, ammonia gas and hydrogen gas in the presence of a catalyst to obtain isophorone diamine; subjecting isophorone diamine to the phosgenation reaction to obtain isophorone diisocyanate, wherein the content of secondary amine group-containing impurities in the isophorone diamine for the phosgenation reaction is less than or equal to 0.5 wt%. The method effectively reduces the content of hydrolyzed chlorine in the isophorone diisocyanate product and the chromaticity of the product.

In the preparation methods of isocyanates disclosed in related art, the color of isocyanate is improved to a certain extent by controlling the polarographic value of amine, the content of alcohol-based impurity in amine, the content of secondary amine-containing impurity in amine, and the content of impurity in phosgene. However, the light-colored isocyanates are prone to darken in color during the subsequent modification and production of polyurethane products, resulting in severe weather resistance issues in the obtained polyurethane products. Long-term use can lead to significant yellowing, thus adversely affecting the appearance and the usability of the polyurethane products, especially polyurethane coating materials and coating layers.

Therefore, the development of isocyanates with excellent performances to improve the discoloration resistance of polyurethane products, especially polyurethane coating materials and coating layers, is a research focus in the field.

SUMMARY

The following is a summary of the subject matters described in detail herein. This summary is not intended to limit

the scope of the claims.

Embodiments of the present application provide an isocyanate composition and a preparation method and use thereof. Through designing and controlling an effective factor, the isocyanate composition can be used to prepare a high-performance polyurethane product, thereby significantly improving the discoloration resistance of the polyurethane product, especially a polyurethane coating material and a coating layer thereof.

In a first aspect, embodiments of the present application provide an isocyanate composition, wherein an effective factor of the isocyanate composition is in a range from 3.70 to 4.70;

the effective factor is calculated according to Formula I:

$$E = -\lg\left(A - \frac{B \times M_{Cl}}{M_B}\right) \quad \text{Formula I;}$$

in Formula I, E is the effective factor;
in Formula I, A is a mass content of chlorine in the isocyanate composition;
in Formula I, B is a mass content of chlorinated isocyanate in the isocyanate composition;
in Formula I, $M_{C1}$ is a relative atomic mass of chlorine; and
in Formula I, $M_B$ is a relative molecular mass of the chlorinated isocyanate.

The effective factor E of the isocyanate composition provided herein can be in a range from 3.70 to 4.70, for example, 3.75, 3.80, 3.85, 3.90, 3.95, 4.00, 4.05, 4.10, 4.15, 4.20, 4.25, 4.30, 4.35, 4.40, 4.45, 4.50, 4.55, 4.60, or 4.65, as well as specific point values between any two of the above point values. The specific point values included within the range are no longer listed exhaustively herein due to the limitation of space and for concise consideration.

**In** the present application, the isocyanate composition includes a combination of an isocyanate and a chlorine-containing substance, and is therefore defined as a "composition". The chlorine-containing substance includes a combination of a chlorinated isocyanate and a substance corresponding to the effective factor. Through the design and control of the effective factor, the isocyanate composition includes a specific kind of chlorine-containing substance with a specific content, which enables the isocyanate composition to have excellent reactivity and to be used for the preparation of a high-performance polyurethane product. The isocyanate composition can effectively improve the discoloration resistance and stability of the polyurethane product, especially making the polyurethane coating material to have excellent discoloration resistance, and maintain excellent color stability under a high temperature and high humidity environment, and thus significantly improving the yellowing resistance and appearance of the coating layer. If the effective factor of the isocyanate composition is too high or too low, the discoloration resistance of the polyurethane coating material can be reduced, and the coating layer can appear significant yellowing under a high temperature and high humidity condition.

In the present application, in formula I for calculating the effective factor, A is the mass content of chlorine in the isocyanate composition. The A is determined by X-ray fluorescence spectroscopy (XRF).

In the present application, in formula I for calculating the effective factor, B is the mass content of chlorinated isocyanate in the isocyanate composition. Preferably, the B is determined by chromatography-mass spectrometry, further preferably by gas chromatography-mass spectrometry (GCMS).

In the study of the present application, it is found that the method for characterization of the content of chlorine in isocyanate disclosed in related art cannot accurately control the performance of isocyanate, and thus cannot effectively control the quality of polyurethane products, especially the discoloration resistance of polyurethane coating materials. Specifically, the method for measuring a total chlorine content under the standard GB/T 12009.1-1989 is an oxygen bottle combustion method, which involves converting all chlorine (including bromine) in isocyanate into inorganic chlorine (including bromine), and then titrating with silver nitrate to characterize the total chlorine content (including the bromine content) in the isocyanate. The Standard GB/T 12009.2-2016 detects hydrolyzed chlorine, which is specifically the chlorine released after the reaction of isocyanate with alcohol and water, and is highly active chlorine (including highly active bromine) in the isocyanate. Monochlorinated isocyanate can also partly be hydrolyzed. The content of chlorine (including a part of bromine) detected by the GB/T 12009.1-1989 or the GB/T 12009.2-2016 cannot accurately indicate the component information of isocyanate, and thus the performances of the isocyanate and polyurethane product thereof cannot be effectively controlled.

As a preferred technical solution of the present application, in the calculation of the effective factor E, A is a total chlorine content (excluding bromine) determined by the XRF test, B is a content of chlorinated isocyanate determined by chromatography-mass spectrometry. The value of A and the value of B are determined by accurate qualitative and

quantitative analysis methods, so that the effective factor E accurately characterizes the polychlorinated compound and the partial hydrolyzed chlorine in the isocyanate composition (not including the hydrolyzed chlorine of monochlorinated isocyanate), thereby corresponding to a more precise and specific chlorine content. This chlorine content plays a key role in the activity of the isocyanate and the performance of the polyurethane product (a polyurethane coating material), thus realizing the regulation of the performance of the isocyanate composition, and thereby effectively improving the performance of the polyurethane product prepared accordingly, in particular remarkably improving the discoloration resistance of the polyurethane coating material.

Preferably, the isocyanate is a diisocyanate, further preferably an aliphatic diisocyanate, including a chain aliphatic diisocyanate and/or a cyclic aliphatic diisocyanate (cycloaliphatic diisocyanate).

Preferably, the isocyanate includes any one or a combination of at least two of pentamethylene diisocyanate (PDI), hexamethylene diisocyanate (HDI), dicyclohexylmethane diisocyanate (HMDI), or isophorone diisocyanate (IPDI).

In the present application, unless otherwise specified, the isocyanates listed include all isomers thereof. For example, dicyclohexylmethane diisocyanate (HMDI) is

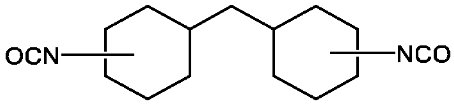

Preferably, a mass content percentage of isocyanate in the isocyanate composition is greater than or equal to 97%, for example, 97.5%, 98%, 98.5%, 99%, 99.1%, 99.2%, 99.3%, 99.4%, 99.5%, 99.6%, 99.7%, 99.8%, 99.9%, 99.92%, 99.95%, 99.98%, 99.99%, etc., and further preferably greater than or equal to 99%.

Preferably, a substance corresponding to the effective factor includes any one or a combination of at least two of following compounds:

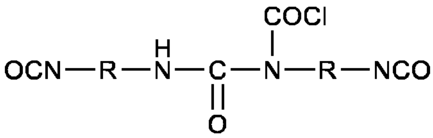

,

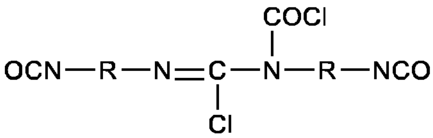

, or

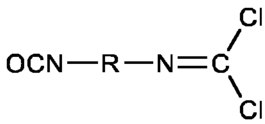

,

where R is a divalent group obtained after removing NCO group in the isocyanate.

Preferably, R is selected from any one or a combination of at least two of linear or branched C6 alkylene (e.g.,

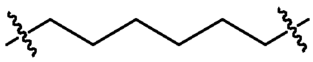

,

isocyanate being HDI), linear or branched C5 alkylene (e.g.,

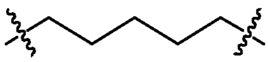

,

isocyanate being PDI),

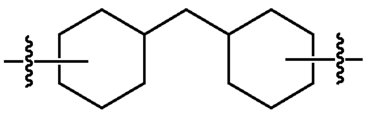

(isocyanate being HMDI), or

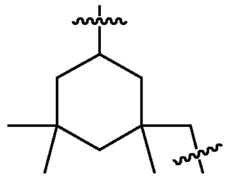

(isocyanate being IPDI), where a wavy line represents an attachment site of the group.

Preferably, the chlorinated isocyanate is a compound obtained by replacing one NCO group in the isocyanate with chlorine.

Preferably, the chlorinated isocyanate includes any one or a combination of at least two of OCN-$R^1$-Cl (chlorohexyl isocyanate CHI, with isocyanate being HDI), OCN-$R^2$-Cl (chloropentyl isocyanate CPI, with isocyanate being PDI),

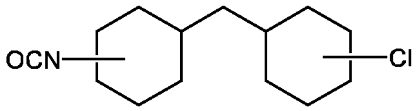

(chlorodicyclohexylmethane isocyanate CHMI, with isocyanate being HMDI),

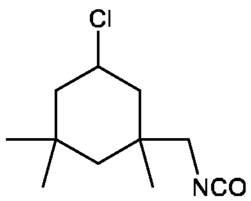 , 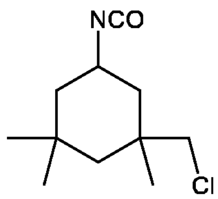

(chloroisophorone diisocyanate CIPI, with isocyanate being IPDI), where $R^1$ is a linear or branched C6 alkylene (e.g.,

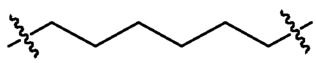 ),

and $R^2$ is a linear or branched C5 alkylene (e.g.,

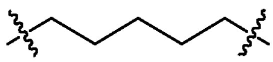 ).

Preferably, the chlorinated isocyanate includes any one or a combination of at least two of

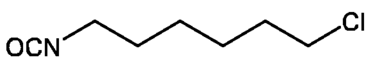 , 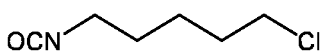 ,

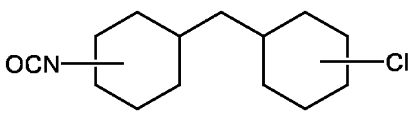 , 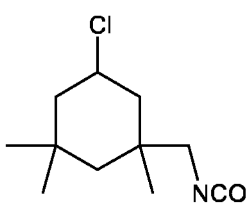 , or 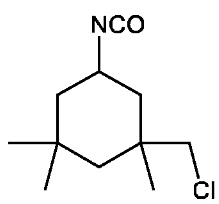 .

Herein, the expression of a ring structure crossed by "-" indicates that an attachment site is at any position on the ring structure where a bond can be formed.

Preferably, the mass content (the value of A) of chlorine in the isocyanate composition is in a range from 2 ppm to 1000 ppm, for example, 5 ppm, 10 ppm, 50 ppm, 100 ppm, 150 ppm, 200 ppm, 250 ppm, 300 ppm, 350 ppm, 400 ppm, 450 ppm, 500 ppm, 550 ppm, 600 ppm, 650 ppm, 700 ppm, 750 ppm, 800 ppm, 850 ppm, 900 ppm, or 950 ppm, as well as specific point values between any two of the above values. The specific point values included within the range are no longer

listed exhaustively herein due to the limitation of space and for concise consideration. The mass content (the value of A) of chlorine in the isocyanate composition is further preferably in a range from 20 ppm to 900 ppm.

Preferably, the mass content (the value of B) of the chlorinated isocyanate in the isocyanate composition is in a range from 5 ppm to 3000 ppm, for example, 20 ppm, 50 ppm, 100 ppm, 300 ppm, 500 ppm, 700 ppm, 900 ppm, 1000 ppm, 1100 ppm, 1300 ppm, 1500 ppm, 1700 ppm, 1900 ppm, 2000 ppm, 2100 ppm, 2300 ppm, 2500 ppm, 2700 ppm, or 2900 ppm, as well as specific point values between any two of the above values. The specific point values included within the range are no longer listed exhaustively herein due to the limitation of space and for concise consideration.

In the present application, "ppm" means parts per million. 1 ppm means one part per million. Hereinafter, the same expression has the same meaning.

It should be understood that, in the present application, the substance corresponding to the effective factor and the chlorinated isocyanate can be produced as by-products during the preparation of isocyanate, or can be artificially added to obtain a desired content.

In a second aspect, embodiments of the present application provide a method for preparing the isocyanate composition as described in the first aspect, including reacting an amine compound with phosgene to obtain the isocyanate composition.

Preferably, the preparation method includes the steps of:

(1) reacting the amine compound with phosgene to obtain a reaction product;
(2) subjecting the reaction product obtained in step (1) to a removal treatment to obtain a crude product, wherein the removal treatment includes a phosgene removal treatment and/or a solvent removal treatment; and
(3) sequentially separating and refining the crude product obtained in step (2) to obtain the isocyanate composition.

Preferably, in step (3), a heavy fraction and an intermediate fraction are obtained by the separation; a mixture of the intermediate fraction and the heavy fraction is refined to obtain the isocyanate composition; and a mass content percentage of the heavy fraction in the mixture is in a range from 1% to 10%.

As a preferred technical solution of the present application, the components undergoing refining is a mixture of the intermediate fraction and the heavy fraction. The mass content percentage of the heavy fraction in the mixture is in a range from 1% to 10%, for example, 2%, 3%, 4%, 5%, 6%, 7%, 8% or 9%, as well as specific point values between any two of the above point values. The specific point values included within the range are no longer listed exhaustively herein due to the limitation of space and for concise consideration. The mass content percentage of the heavy fraction in the mixture is further preferably in a range from 2% to 10%.

Preferably, the separated heavy fraction can be directly mixed with the intermediate fraction to obtain the mixture. Alternatively, the separated heavy fraction is a primary heavy fraction, and the primary heavy fraction is separated again to obtain a heavy fraction recovered material and a residual heavy fraction. The heavy fraction recovered material is mixed with the intermediate fraction to obtain the mixture. The mass content percentage of the heavy fraction recovered material in the mixture is in a range from 1% to 10%.

In another preferred technical solution, the method for preparing the isocyanate composition includes mixing the isocyanate obtained by pyrolysis of carbamate and the heavy fraction recovered material to obtain the isocyanate composition. Preferably, the mass content percentage of the heavy fraction recovered material in the isocyanate composition is in a range from 1% to 10%, e.g., 2%, 3%, 4%, 5%, 6%, 7%, 8%, 9%, etc., and further preferably in a range from 1% to 5%.

As a preferred technical solution of the present application, the method for preparing the isocyanate composition is a phosgenation method, that is, an amine compound reacts with phosgene to prepare isocyanate. Such amine compound includes diamine and/or a diamine salt, e.g., a diamine hydrochloride obtained by reacting the diamine with HCl.

Preferably, the method of reacting the amine compound with phosgene exemplarily includes the following three categories: a method of reacting diamine with phosgene in a gas phase, also known as a gas phase phosgenation method; a method of reacting diamine with phosgene in a liquid phase, also known as a liquid phase phosgenation method; and a method of reacting a diamine salt (e.g., diamine hydrochloride) with phosgene in a solvent, also known as a diamine hydrochloride phosgenation method; and further preferably the gas phase phosgenation method.

Preferably, the reaction in step (1) is carried out in a reaction zone with or without an inert medium.

Preferably, the reaction in step (1) is carried out in a gas phase, i.e., the gas phase phosgenation method. The amine compound (diamine) is vaporized in advance, and the resulting diamine in a gas phase form is free of droplets before entering the reaction zone.

Preferably, the reaction in step (1) can be carried out in a batch operation, a semi-continuous operation, or a continuous operation, further preferably in a continuous operation.

Preferably, the inert medium is selected from any one or a combination of at least two of nitrogen gas, noble gases (e.g., argon gas and/or helium gas), aromatic compounds (e.g., chlorobenzene, dichlorobenzene, toluene, xylene),

carbon monoxide, carbon dioxide or the like, further preferably any one or a combination of at least two of nitrogen gas, chlorobenzene, or dichlorobenzene.

Preferably, the amount of the inert medium used is calculated by a gas volume of the vaporized inert medium. The volume ratio of the inert medium (gas) to the diamine (gas) is in a range from 0.01:1 to 5:1, for example, 0.02:1, 0.05:1, 0.08:1, 0.1:1, 0.3:1, 0.5:1, 0.8:1, 1:1, 1.5:1, 2:1, 2.5:1, 3:1, 3.5:1, 4:1, or 4.5:1, further preferably 0.05:1 to 3:1, more preferably 0.1:1 to 1:1.

Preferably, the reaction in step (1) specifically includes reacting the vaporized diamine with phosgene in a reaction zone to obtain a reaction product.

Preferably, a molar ratio of phosgene to the amine compound (diamine) is in a range from 2.5:1 to 20:1, for example, 3:1, 5:1, 7:1, 9:1, 10:1, 11:1, 13:1, 15:1, 17:1, 19:1, etc., preferably 4:1 to10:1, and more preferably 6:1 to 10:1.

Preferably, the temperature of the reaction in step (1) is in a range from 300°C to 500°C, for example, 310°C, 330°C, 350°C, 370°C, 390°C, 400°C, 410°C, 430°C, 450°C, 470°C, or 490°C, as well as specific point values between any two of the above point values. The specific point values included within the range are no longer listed exhaustively herein due to the limitation of space and for concise consideration. The temperature of the reaction in step (1) is further preferably in a range from 350°C to 450°C.

Preferably, the absolute pressure of the reaction in step (1) is in a range from 0.05 MPa to 0.3 MPa, for example, 0.06 MPa, 0.08 MPa, 0.1 MPa, 0.12 MPa, 0.15 MPa, 0.18 MPa, 0.2 MPa, 0.22 MPa, 0.25 MPa, or 0.28 MPa, as well as specific point values between any two of the above point values. The specific point values included within the range are no longer listed exhaustively herein due to the limitation of space and for concise consideration. The absolute pressure of the reaction in step (1) is further preferably in a range from 0.07 MPa to 0.2 MPa, and more preferably 0.09 MPa to 0.18 MPa.

Preferably, flow rates of the vaporized diamine feed stream and the phosgene feed stream into the reaction zone are each independently in a range from 5 m/s to 100 m/s, for example, 10 m/s, 15 m/s, 20 m/s, 25 m/s, 30 m/s, 35 m/s, 40 m/s, 45 m/s, 50 m/s, 55 m/s, 60 m/s, 65 m/s, 70 m/s, 75 m/s, 80 m/s, 85 m/s, 90 m/s, or 95 m/s, as well as specific point values between any two of the above point values. The specific point values included within the range are no longer listed exhaustively herein due to the limitation of space and for concise consideration. Further preferably, the flow rates of the vaporized diamine feed stream and the phosgene feed stream into the reaction zone are each independently in a range from 10 m/s to 80 m/s.

Preferably, the average contact time between the amine compound (diamine) and the phosgene in the reaction zone is in a range from 0.01 s to 15 s, for example, 0.02 s, 0.05 s, 0.08 s, 0.1 s, 0.3 s, 0.5 s, 0.8 s, 1 s, 2 s, 3 s, 4 s, 5 s, 6 s, 7 s, 8 s, 9 s, 10 s, 11 s, 12 s, 13 s, or 14 s, as well as specific point values between any two of the above point values. The specific point values included within the range are no longer listed exhaustively herein due to the limitation of space and for concise consideration. Further preferably, the average contact time between the amine compound (diamine) and the phosgene in the reaction zone is in a range from 0.04 s to 10 s, and more preferably 0.08 s to 5 s.

Preferably, a reaction product of the amine compound (diamine) and phosgene in step (1) is spray washed (i.e., trapped) by using an inert solvent in a single stage or multiple stages to reduce the temperature of the product to be equal to or less than 150°C, obtaining the reaction product, i.e., a reaction solution containing isocyanate.

Preferably, the inert solvent is an organic solvent. Examples thereof include but are not limited to: aromatic hydrocarbons such as benzene, toluene, and xylene, aliphatic hydrocarbons such as octane and decane, alicyclic hydrocarbons such as cyclohexane, methylcyclohexane, and ethylcyclohexane, halogenated aromatic hydrocarbons such as chlorotoluene, chlorobenzene, dichlorobenzene, dibromobenzene, and trichlorobenzene, nitrogen-containing compounds such as nitrobenzene, *N,* N-dimethylformamide, *N, N*-dimethylacetamide, and *N*, N'-dimethylimidazolinone, ethers such as dibutyl ether, ethylene glycol dimethyl ether, and ethylene glycol diethyl ether, ketones such as heptanone, diisobutyl ketone, methyl isobutyl ketone, and methyl ethyl ketone, fatty acid esters such as ethyl acetate, butyl acetate, amyl acetate, and ethoxyethyl acetate, and aromatic carboxylic acid esters such as methyl salicylate, dimethyl phthalate, dibutyl phthalate, and methyl benzoate. The inert solvent can be used alone or as a combination of at least two solvents.

Preferably, the inert solvent includes halogenated aromatic hydrocarbons, further preferably chlorobenzene and/or dichlorobenzene.

If necessary, the reaction product obtained in step (1) can be subjected to a removal process (including a solvent removal process and/or a phosgene removal process) and separating and refining processes.

Preferably, the phosgene removal treatment in step (2) is performed in a phosgene removal tower.

Preferably, the solvent removal treatment in step (2) is performed in a solvent removal tower.

Preferably, the separation in step (3) is performed to separate a heavy fraction from an intermediate fraction (i.e., a light fraction), so as to achieve the removal of the heavy fraction. The apparatus for the separation exemplarily includes, but is not limited to, a short-path evaporator, a distillation tower.

Preferably, the operation pressure of the short-path evaporator is in a range from 0.05 kPa to 4 kPa, for example, 0.08 kPa, 0.1 kPa, 0.3 kPa, 0.5 kPa, 0.8 kPa, 1 kPa, 1.2 kPa, 1.5 kPa, 1.8 kPa, 2 kPa, 2.2 kPa, 2.5 kPa, 2.8 kPa, 3 kPa, 3.2 kPa, 3.5 kPa or 3.8 kPa, as well as specific point values between any two of the above point values. The specific point values included within the range are no longer listed exhaustively herein due to the limitation of space and for concise

consideration. Further preferably, the operation pressure of the short-path evaporator is in a range from 0.1 kPa to 2.5 kPa.

As a preferred technical solution of the present application, the heavy fraction obtained by the separation contains chlorine-containing substances with relatively rich kinds and relatively high contents. The heavy fraction or the heavy fraction recovered material obtained by re-separation of the heavy fraction is mixed into the separated intermediate fraction (the light fraction) in a certain proportion, and then the mixture is refined, so that kinds and contents of the chlorine-containing substances in the product can be effectively controlled to obtain the effective factor of the isocyanate composition of 3.90 to 5.70.

Preferably, the mass content percentage of the heavy fraction (the heavy fraction recovered material) in the mixture (i.e., materials to be refined) is in a range from 1% to 10%, further preferably 2% to 10%, so that the effective factor of the isocyanate composition can be in a range from 3.70 to 4.70. If the amount of the heavy fraction (the heavy fraction recovered material) added is too small, the effective factor is relatively high, so that when used to prepare polyurethane products, the isocyanate composition will lead to an excessively fast reaction rate and poor discoloration resistance. If the amount of the heavy fraction (the heavy fraction recovered material) added is too high, the effective factor is relatively low, so that the isocyanate composition contains relatively more impurities, which will also affect the discoloration resistance and stability of polyurethane products, resulting in significantly yellowing of polyurethane coating materials in a hot and humid environment.

Preferably, the heavy fraction mixed with the intermediate fraction can be added directly into the intermediate fraction. Alternatively, the heavy fraction can be recovered and separated through a heavy fraction removal apparatus to obtain a heavy fraction recovered material, which is then added into the intermediate fraction.

Preferably, the method for refining is an industrial separation technique known in the art, and exemplarily includes, but is not limited to, distillation, rectification, crystallization, etc.

Preferably, the refining process in step (3) is rectification.

Preferably, the rectification is carried out in a rectification tower, which preferably includes a plate rectification tower or a packed rectification tower.

Preferably, the theoretical number of plates in the rectification tower is in a range from 2 to 60, for example, 3, 5, 8, 10, 12, 15, 18, 20, 22, 25, 28, 30, 32, 35, 38, 40, 42, 45, 48, 52, 52, 55, or 58, as well as specific point values between any two of the above point values. The specific point values included within the range are no longer listed exhaustively herein due to the limitation of space and for concise consideration. Further preferably, the theoretical number of plates in the rectification tower is in a range from 5 to 40.

Preferably, the pressure at the top of the rectification tower is in a range from 0.1 kPa to 4 kPa, for example, 0.2 kPa, 0.5 kPa, 0.8 kPa, 1 kPa, 1.2 kPa, 1.5 kPa, 1.8 kPa, 2 kPa, 2.2 kPa, 2.5 kPa, 2.8 kPa, 3 kPa, 3.2 kPa, 3.5 kPa, or 3.8 kPa, as well as specific point values between any two of the above point values. The specific point values included within the range are no longer listed exhaustively herein due to the limitation of space and for concise consideration. Further preferably, the pressure at the top of the rectification tower is in a range from 0.15 kPa to 2.5 kPa.

Preferably, the reflux ratio at the top of the rectification tower is in a range from 0.01 to 60, for example, 0.05, 0.1, 0.5, 1, 3, 5, 8, 10, 12, 15, 18, 20, 22, 25, 28, 30, 32, 35, 38, 40, 42, 45, 48, 50, 52, 55, or 58, as well as specific point values between any two of the above point values. The specific point values included within the range are no longer listed exhaustively herein due to the limitation of space and for concise consideration. Further preferably, the reflux ratio at the top of the rectification tower is in a range from 0.1 to 40.

In a preferred technical solution of the present application, the method for preparing the isocyanate composition includes the following steps of:

(1) a phosgenation process: reacting a vaporized diamine with phosgene, and spray washing (trapping) a resulting product with an inert solvent to obtain a reaction product;
(2) a removal process: subjecting the reaction product obtained in step (1) to a removal treatment to obtain a crude product, wherein the removal treatment includes a phosgene removal treatment and/or a solvent removal treatment;
(3a) a separation process: separating the crude product obtained in step (2) to obtain a heavy fraction and an intermediate fraction (a light fraction);
(3b) a heavy fraction recovering process: mixing the intermediate fraction and the heavy fraction that are obtained in step (3a) to obtain a mixture, wherein the mass content percentage of the heavy fraction in the mixture is in a range from 1% to 10%; or subjecting the heavy fraction obtained in step (3a) to secondary separation to obtain a heavy fraction recovered material and a residual heavy fraction, and mixing the heavy fraction recovered material with the intermediate fraction to obtain a mixture, wherein the mass content percentage of the heavy fraction recovered material in the mixture is in a range from 1% to 10%; and
(3c) a refining process: refining the mixture obtained in step (3b) to obtain the isocyanate composition.

Exemplarily, a schematic flow chart of the preparation method is shown in FIG. 1, including a phosgenation process 10, a removal process 20, a separation process 30, a heavy fraction recovering process 40, and a refining process

50. The phosgenation process can be performed in a batch mode or a continuous mode. The effective factor of the isocyanate composition can be adjusted by appropriately adjusting the mixing ratio of the heavy fraction to the intermediate fraction, the ratio of phosgene supply, the reaction temperature, the reflux ratio of the rectification tower, and the like, in which the effective factor is mainly controlled by the mixing ratio of the heavy fraction to the intermediate fraction.

Specifically, taking an HDI composition as an example, the preparation method is as follows.

(1) Phosgenation process: Vaporized 1, 6-hexamethylenediamine optionally mixed with nitrogen gas is continuously reacted with phosgene in a tubular reactor; and after leaving the reaction zone, the resulting product is continuously spray washed (trapped) with chlorobenzene to obtain a reaction product, that is, a reaction solution containing isocyanate.

Thus, the phosgenation process is continuously performed.

Thus, the salt-forming process and the phosgenation process are continuously performed.

(2) Removal process: A phosgene removal tower and a solvent removal tower are used. The reaction solution as obtained above is continuously introduced into a middle portion of the phosgene removal tower, in which phosgene, hydrogen chloride and the like are removed from the reaction solution through the phosgene removal tower. The solvent is removed from the reaction solution through the solvent removal tower to obtain an HDI crude product.

(3a) Separation process: The HDI crude product is separated through a short-path evaporator to remove a heavy fraction, so as to obtain an intermediate fraction and a primary heavy fraction.

(3b) Heavy fraction recovering process: The primary heavy fraction is recovered through the short-path evaporator in a single cycle or multiple cycles to obtain a heavy fraction recovered material and a secondary heavy fraction. The mixture obtained by mixing the heavy fraction recovered material and the intermediate fraction enters the refining process, where the mass content percentage of the heavy fraction recovered material in the mixture is in a range from 1% to 10%.

(3c) Refining process: The mixture as described above is introduced continuously into a rectification tower. Then, under the above-described rectification conditions (including the bottom temperature, the top temperature, the top pressure, the bottom reflux ratio, the top reflux ratio, the residence time), a low boiling material is distilled off from the intermediate fraction, and an HDI composition is taken out from the lower-middle part of the tower.

As such, an HDI composition including HDI, CHI, and substances corresponding to the effective factor can be continuously produced.

In a third aspect, embodiments of the present application provide a modified isocyanate composition, wherein the modified isocyanate composition is obtained by modifying the isocyanate composition as described in the first aspect.

The modified isocyanate composition includes any one or a combination of at least two of groups (a) to (i): (a) isocyanurate group, (b) uretdione group, (c) biuret group, (d) carbamate group, (e) ureido group, (f) iminooxadiazinedione group, (g) allophanate group, (h) uretonimine group, or (i) carbodiimide group.

Those skilled in the art can modify the aforementioned isocyanate composition by a known method as necessary to obtain the modified isocyanate composition. The modified isocyanate composition, as an isocyanate-based substance (a polyisocyanate component), and an active hydrogen group-containing substance are suitably used as raw materials for a polymer such as a polyurethane.

Specifically, the modified isocyanate composition including (a) isocyanurate group is a trimer of isocyanate, which can be obtained, for example, by reacting the isocyanate composition in the presence of a known isocyanurate catalyst to isocyanurate the isocyanate therein.

The modified isocyanate composition including (b) uretdione group can be obtained by heating the isocyanate composition at a temperature ranged from 90°C to 200°C, or by reacting the isocyanate composition in the presence of a known uretdionization catalyst to subject the isocyanate to uretdionization (e.g., dimerization).

The modified isocyanate composition including (c) biuret group can be obtained by reacting the isocyanate composition with, for example, water, a tertiary alcohol (e.g., tert-butanol etc.), a secondary amine (e.g., dimethylamine, diethylamine, etc.), or the like, and then further reacting in the presence of a known biuretation catalyst.

The modified isocyanate composition including (d) carbamate group can be obtained by reacting the isocyanate composition with a polyol component such as trimethylolpropane or the like.

The modified isocyanate composition including (e) ureido group can be obtained by reacting the isocyanate composition with water, a polyamine component, or the like.

The modified isocyanate composition including (f) iminooxadiazinedione group is an asymmetric trimer of isocyanate, which can be obtained by reacting the isocyanate composition in the presence of a known iminooxadiazinedionation catalyst to subject the isocyanate to iminooxadiazinedionation (e.g., trimerization).

The modified isocyanate composition including (g) allophanate group can be obtained by reacting the isocyanate composition with an alcohol and then further reacting in the presence of a known allophanate catalyst.

The modified isocyanate composition including (h) uretonimine group can be obtained by reacting the isocyanate

composition to form a carbodiimide group in the presence of a known carbodiimidization catalyst, and then adding an isocyanate to the carbodiimide group.

The modified isocyanate composition including (i) carbodiimide group can be obtained by reacting the isocyanate composition in the presence of a known carbodiimidization catalyst.

It should be noted that, the modified isocyanate composition can contain at least one or two of the above groups (a) to (i). Such modified isocyanate composition can be produced by appropriately combining the above-described reactions. In addition, the modified isocyanate composition alone or in combination of two or more can be used.

Taking the HDI composition as an example, those skilled in the art can modify the HDI composition by a known method as necessary to obtain a modified HDI composition. The modified HDI composition, as an isocyanate-based substance (a polyisocyanate component), and an active hydrogen group-containing substance are suitably used as raw materials of polyurethane.

In a fourth aspect, embodiments of the present application provide an isocyanate-based polymer, wherein the polymer is prepared by reacting an isocyanate-based substance with an active hydrogen group-containing substance. The isocyanate-based substance includes at least one of the isocyanate composition as described in the first aspect or the modified isocyanate composition as described in the third aspect.

Preferably, the active hydrogen group includes any one or a combination of at least two of hydroxyl group, amino group, or sulfhydryl group.

Preferably, the active hydrogen group-containing substance includes any one or a combination of at least two of a polyol, a polyamine, or a polythiol.

When the active hydrogen group-containing substance is a polyol, the polymer is a polyurethane. When the active hydrogen group-containing substance is a polyamine, the polymer is a polyurea. When the active hydrogen group-containing substance is a polythiol, the polymer is a polythiourethane.

In a fifth aspect, embodiments of the present application provide a two-component polyurethane composition, wherein the two-component polyurethane composition includes agent A and agent B. The agent A includes the isocyanate composition as described in the first aspect and/or the modified isocyanate composition as described in the third aspect. The agent B includes the active hydrogen group-containing substance.

The two-liquid type two-component polyurethane composition in which the isocyanate-based substance containing the isocyanate composition and/or the modified isocyanate composition is used as the agent A and the active hydrogen group-containing substance is used as the agent B, can be applied to a coating raw material such as a coating material and an adhesive, a two-component curable sealing material, a potting agent, etc. Such two-component polyurethane composition is a kind of raw material which is obtained by blending the agent A (a curing agent) and the agent B (a main agent) prepared separately before use.

The coating raw material is a two-component curable resin raw material for forming a coating layer, and contains the agent A (a curing agent) and the agent B (a main agent). The coating layer can contain a coating material, an adhesive, etc.

When the coating raw material is used as the coating material, the uses can exemplarily include, but are not limited to: coating materials for plastics, coating materials for automobile exterior decoration, coating materials for automobile interior decoration, coating materials for electrical/electronic materials, coating materials for optical materials (lenses, etc.), coating materials for building materials, glass coating materials, woodworking coating materials, film coating materials, ink coating materials, coating materials (coating layer agents) for artificial leather , coating materials (coating layer agents) for cans, etc.

Preferably, the agent A includes the modified isocyanate composition, which is obtained by modifying the isocyanate composition and preferably includes (a) isocyanurate group and/or (d) carbamate group.

In addition, the agent A can further contain other aromatic isocyanates, other aliphatic isocyanates, or other aromatic aliphatic isocyanates as necessary.

In the present application, the agent A in the two-component polyurethane composition includes the isocyanate composition and/or the modified isocyanate composition. The effective factor of the isocyanate composition is in a range from 3.70 to 4.70. The modified isocyanate composition is obtained by modifying the isocyanate composition having the effective factor ranged from 3.70 to 4.70. Through the design and control of the effective factor, the two-component polyurethane composition as a two-component polyurethane coating material can effectively inhibit the discoloration of the coating, and enable the coating to maintain excellent stability in a high-temperature and high-humidity environment, significantly improving the discoloration resistance of the coating.

Preferably, the active hydrogen group in the agent B includes any one or a combination of at least two of hydroxyl group, amino group, or sulfhydryl group (thiol group).

Preferably, the agent B includes any one or a combination of at least two of a polyol (a component containing at least 2 hydroxyl groups), a polythiol (a component containing at least 2 sulfhydryl groups /thiol groups), or a polyamine (a component containing at least 2 amino groups)

Preferably, the agent B includes a polyol.

Preferably, the polyol includes a low molecular weight polyol and/or a high molecular weight polyol.

Preferably, the low molecular weight polyol is a compound containing at least 2 hydroxyl groups and having a number average molecular weight ranged from 60 to 400, e.g., 80, 100, 150, 200, 250, 300, 350, etc.

Exemplarily, the low molecular weight polyol includes, for example, diols such as ethylene glycol, propylene glycol, 1,3-propanediol, 1,4-butanediol, 1,3-butanediol, 1,2-butanediol, 1,5-pentanediol, 1,6-hexanediol, neopentyl glycol, C7-C22 alkane diol, diethylene glycol, triethylene glycol, dipropylene glycol, 3-methyl-1,5-pentanediol, C17-C20 alkane-1,2-diol, isosorbide, 1,3-cyclohexanedimethanol, 1,4-cyclohexanedimethanol, 1,4-cyclohexanediol, hydrogenated bisphenol A, 1,4-dihydroxy-2-butene, 2,6-dimethyl-1-octene-3,8-diol, and bisphenol A; triols such as glycerin and trimethylolpropane; tetrols such as tetramethylolmethane (pentaerythritol) and diglycerin; pentols such as xylitol; hexols such as sorbitol, mannitol, allitol, iditol, dulcitol, altritol, inositol, and dipentaerythritol; heptols such as persitol; octols such as sucrose, and the like.

In addition, a polyalkylene oxide, as a random and/or block copolymer containing at least two kinds of alkylene oxides, having a number average molecular weight of 60 to 400 and obtained by an addition reaction of alkylene oxides such as ethylene oxide and propylene oxide with the above-described alcohol as an initiator, is also included in the low molecular weight polyol.

Preferably, the high molecular weight polyol is a compound containing at least 2 hydroxyl groups and having a number average molecular weight ranged from 400 to1000, e.g., 500, 800, 1000, 2000, 3000, 4000, 5000, 6000, 7000, 8000, 9000, etc. Further preferably, the number average molecular weight of the high molecular weight polyol is in a range from 400 to 5000.

Preferably, the high molecular weight polyol includes any one or a combination of at least two of a polyether polyol, a polyester polyol, a polycarbonate polyol, a polyurethane polyol, an epoxy polyol, a vegetable oil polyol, a polyolefin polyol, an acrylic polyol, a polysiloxane polyol, a fluoropolyol, or a vinyl monomer modified polyol.

Exemplarily, the polyether polyol includes polyoxy (C2-C3) alkylene polyol, polytetramethylene ether glycol, polytrimethylene ether glycol, etc. An example of the polyoxy (C2-C3) alkylene polyol includes a polymer (a random and/or block copolymer containing at least two kinds of alkylene oxides) obtained by an addition reaction of C2-C3 alkylene oxides such as ethylene oxide and propylene oxide with the above-described low molecular weight polyol as an initiator. Specifically, the polyoxy (C2-C3) alkylene polyol includes, for example, polyethylene glycol, polypropylene glycol, polyethylene-polypropylene copolymer, and the like. Examples of the polytetramethylene ether glycol include a ring-opening polymer (i.e., polytetramethylene ether glycol) obtained by cationic polymerization of tetrahydrofuran, and an amorphous polytetramethylene ether glycol obtained by copolymerizing a polymerized unit of tetrahydrofuran with the above-described diols. In addition, an example thereof further includes plant-derived polytetramethylene ether glycol using tetrahydrofuran produced from plant-derived raw materials such as furfural as a starting material. Examples of the polytrimethylene ether glycol include polyols produced by polycondensation of plant-derived 1,3-propanediol.

Exemplarily, the polyester polyol includes a polycondensate obtained by reacting the above-described low molecular weight polyol preferably a diol with a polybasic acid preferably a dibasic acid under a known condition.

Examples of the polybasic acid include saturated aliphatic dicarboxylic acids (C11-C13) such as oxalic acid, malonic acid, succinic acid, methylsuccinic acid, glutaric acid, adipic acid, 1,1-dimethyl-1,3-dicarboxypropane, 3-methyl-3-ethylglutaric acid, azelaic acid, and sebacic acid; unsaturated aliphatic dicarboxylic acids such as maleic acid, fumaric acid, and itaconic acid; aromatic dicarboxylic acids such as phthalic acid, isophthalic acid, terephthalic acid, toluene dicarboxylic acid, and naphthalene dicarboxylic acid; alicyclic dicarboxylic acids such as hexahydrophthalic acid; other carboxylic acids such as dimer acids, hydrogenated dimer acids, and HET acids; anhydrides derived from the aforementioned carboxylic acids, such as oxalic anhydride, succinic anhydride, maleic anhydride, phthalic anhydride, 2-alkyl (C12-C18) succinic anhydride, tetrahydrophthalic anhydride, and trimellitic anhydride; and acid halides derived from these carboxylic acids, such as oxalyl dichloride, adipic dichloride, sebacic dichloride, and the like.

In addition, examples of the polyester polyol include vegetable oil-based polyester polyols obtained by subjecting the above-described low molecular weight polyol to a condensation reaction with a hydroxyl group-containing vegetable oil fatty acid (for example, a hydroxycarboxylic acid such as a castor oil fatty acid containing ricinoleic acid and a hydrogenated castor oil fatty acid containing 12-hydroxystearic acid) under a known condition.

In addition, examples of the polyester polyol include for example, polycaprolactone polyol and polyvalerolactone polyol obtained by ring-opening polymerization of lactones such as ε-caprolactone and γ-valerolactone with the above-described low molecular weight polyols preferably a diol as an initiator, and lactone-based polyester polyol obtained by copolymerizing these with the above-described diols.

Exemplarily, examples of the polycarbonate polyol include ring-opening polymers of ethylene carbonate using the above-described low molecular weight polyol preferably a diol as an initiator, and amorphous polycarbonate polyols obtained by copolymerizing the above-described diols and the ring-opening polymers.

Exemplarily, examples of the polyurethane polyol include for example, polyester polyurethane polyol, polyether polyurethane polyol, polycarbonate polyurethane polyol, polyester polyether polyurethane polyol, etc. obtained by reacting the polyester polyol, the polyether polyol, and/or the polycarbonate polyol obtained in the above-described

method with the polyisocyanate (including HDI, hereinafter) at an equivalent ratio (OH/NCO) of hydroxyl groups to isocyanate groups being greater than 1.

Exemplarily, examples of the epoxy polyol include epoxy polyols obtained by reacting the above-described low molecular weight polyol with a polyfunctional halohydrin such as epichlorohydrin and β-methyl epichlorohydrin.

Exemplarily, examples of the vegetable oil polyol include hydroxyl-containing vegetable oils such as castor oil and coconut oil. Further, examples thereof also include a castor oil polyol, or an ester-modified castor oil polyol obtained by reacting the castor oil polyol with polypropylene polyol.

Exemplarily, examples of the polyolefin polyol include polybutadiene polyols, partially saponified ethylene-vinyl acetate copolymers, and the like.

Exemplarily, examples of the acrylic polyol include copolymers obtained by copolymerizing hydroxyl group-containing acrylate and a copolymerizable vinyl monomer which can be copolymerized with the hydroxyl group-containing acrylate.

Examples of the hydroxyl group-containing acrylate include 2-hydroxyethyl(methyl)acrylate, hydroxypropyl(methyl)acrylate, hydroxybutyl(methyl)acrylate, 2,2-dihydroxymethylbutyl(methyl)acrylate, polyhydroxyalkyl maleate, polyhydroxyalkyl fumarate, etc., preferably, 2-hydroxyethyl(methyl)acrylate, and the like.

Examples of the copolymerizable vinyl monomer include alkyl (methyl)acrylate (C1-C12) such as methyl(methyl) acrylate, ethyl(methyl)acrylate, propyl(methyl)acrylate, isopropyl(methyl)acrylate, butyl(methyl)acrylate, isobutyl (methyl)acrylate, sec-butyl(methyl)acrylate, tert-butyl(methyl)acrylate, pentyl(methyl)acrylate, isopentyl(methyl)acrylate, hexyl(methyl)acrylate, isononyl(methyl)acrylate, 2-ethylhexyl(methyl)acrylate, cyclohexylacrylate, and isobornyl(methyl)acrylate; aromatic vinyl monomers such as styrene, vinyl toluene, and α-methyl styrene; vinyl cyanide such as (methyl)acrylonitrile; vinyl monomers having carboxyl group such as (methyl)acrylic acid, fumaric acid, maleic acid, itaconic acid, or alkyl esters thereof; alkyl polyol poly(methyl)acrylate such as ethylene glycol di(methyl)acrylate, butylene glycol di(methyl)acrylate, hexylene glycol di(methyl)acrylate, oligoethylene glycol di(methyl)acrylate, trimethylol propane di(methyl)acrylate, and trimethylol propane tris(methyl)acrylate; and vinyl monomers containing isocyanate group such as 3-(2-isocyanato-2-propyl)-α-methyl styrene, etc.

Further, the acrylic polyols can be obtained by copolymerizing the hydroxyl group-containing acrylates and the copolymerizable vinyl monomers in the presence of appropriate solvents and polymerization initiators.

In addition, the acrylic polyol can include a polysiloxane polyol and a fluoropolyol.

Exemplarily, the polysiloxane polyol can be an acrylic polyol obtained by blending a vinyl group-containing polysiloxane compound such as γ-methacryloxypropyltrimethoxysilane as a copolymerizable vinyl monomer in copolymerization of the acrylic polyol as described above.

Exemplarily, the fluoropolyol can be an acrylic polyol obtained by blending a vinyl group-containing fluorine compound such as tetrafluoroethylene and chlorotrifluoroethylene as a copolymerizable vinyl monomer in the copolymerization of the acrylic polyol as described above.

Exemplarily, the vinyl monomer-modified polyol can be obtained by reacting the above-described high molecular weight polyol with the vinyl monomer such as the above-described alkyl(methyl)acrylate.

The above-described polyol components can be used alone or in combination of two or more.

In addition, as necessary, appropriate proportions of a carbamate catalyst, a hydrolysis inhibitor, a defoamer, a surfactant, a sliding modifier, a surface conditioner, an antioxidant, a weather resistance stabilizer, a pigment, a dye, a filler, a resin powder, etc., can be blended in the agent B.

Preferably, a method for forming the two-component polyurethane composition as a coating material includes, for example, mixing the agent A and the agent B, coating the mixture liquid to a coating object by a known method, and then curing, thereby forming the coating material (a coating layer). Such coating material is excellent in discoloration resistance.

As a preferred technical solution of the present application, the two-component polyurethane composition used as a polyurethane coating material can form a coating layer with excellent discoloration resistance, and good color stability at high temperature and high humidity. The color difference Δb of the coating layer after a damp heat durability test (2000 h) is equal to or less than 1.2. For example, Δb can be 1.15, 1.1, 1.05, 1.0, 0.9, 0.8, 0.7, 0.6, 0.5, etc., further preferably less than 1.2.

Compared with related art, the embodiments of the present application have the following beneficial effects: The isocyanate composition provided in the embodiments of the present application has excellent reactivity through the design and control of the effective factor, and can be used for the preparation of high-performance polyurethane products. The isocyanate composition can effectively improve the stability of the polyurethane product, especially make the polyurethane coating material to have excellent discoloration resistance, and maintain excellent color stability in a high temperature and high humidity environment. The color difference Δb of the coating layer after the damp heat durability test of 2000 h is equal to or less than 1.2, significantly improving the yellowing resistance and appearance of the coating layer.

Other aspects can be understood upon reading and understanding the accompanying drawings and detailed description.

BRIEF DESCRIPTION OF THE DRAWINGS

The accompanying drawings are intended to provide a further understanding of the technical solutions herein and constitute a part of the specification. The accompanying drawings and the embodiments of the present application are collectively used to explain the technical solutions herein, and do not constitute a limitation on the technical solutions herein.

FIG. 1 is a schematic flow chart of a method for preparing an isocyanate composition according to a specific embodiment of the present application.

Reference signs:

10: phosgenation process, 20: removal process, 30: separation process, 40: heavy fraction recovering process, 50: refining process.

DETAILED DESCRIPTION

The technical solutions of the present application will be further described below with reference to embodiments. It should be understood to those skilled in the art that the examples are merely for facilitating understanding the present application and should not be construed as specific limitations on the present application.

The methods for testing components and performances in the present application are as follows:

1. A mass content (a value of A) of chlorine in the isocyanate composition is determined by the XRF test.

   Instrument: Energy Dispersive X-Ray Fluorescence Spectroscope (ED-XRF), Model: MERAK-LE II.
   Method: Standard addition method.
   Principle and operation: Element Cl in a sample is excited by the X-ray generated by an X-ray tube to produce characteristic X-ray fluorescence, with the chromatographically pure $CCl_4$ standard substance as a Cl source, and ethyl acetate as a diluent. The intensity of characteristic X-ray fluorescence is linear with the element concentration. A standard curve is established, and an extrapolated value represents the content of element Cl in the sample.

2. A mass content (a value of B) of chlorinated isocyanate in the isocyanate composition is determined by the GCMS test.

   The gas chromatography-mass spectrometry is performed under the following conditions for analysis. The content herein is a normalized content.
   Analytical instrument: Agilent 5977B GCMS.
   Chromatographic column: DB-5 column, size: 30 m×0.25 mm×0.25 μm.
   Column oven temperature: The temperature is held at 50°C for 2 min, raised up to 80°C at a rate of 5 mL/min, continually raised up to 280°C at a rate of 15 mL/min, and then held for 10 min.
   Separation Ratio: No Split.
   Injection port temperature: 280°C.
   Detection temperature: 300°C.
   Carrier gas: Helium gas.
   Flow rate of carrier gas: 1 mL/min, a constant flow rate.
   Injection volume: 1 μL.
   Detection method: SIM selective ion scanning mode (selective ions for HDI: 160/126, selective ions for PDI: 146/112, selective ions for HMDI: 254/220, selective ions for IPDI: 214/180).

3. A mass content percentage of isocyanate in the isocyanate composition is determined by the gas chromatography test.

The gas chromatography is performed under the following conditions for analysis. The content herein is a normalized content.

Analytical instrument: Agilent 7890B GC.
Chromatographic column: DB-5 column, size: 30 m×0.25 mm×0.25 μm.
Column oven temperature: The temperature is held at 50°C for 1 min, raised up to 300°C at a rate of 10°C/min, and

then held for 5 min.
Separation Ratio: 30:1.
Injection port temperature: 280°C.
Detection temperature: 320°C.
Carrier gas: Nitrogen gas.
Flow rate of carrier gas: 1 mL/min, a constant flow rate.
Injection volume: 1 μL.
Detector: FID.

In the following embodiments of the present application, unless otherwise specified, "parts" and "%" are based on mass.

Example 1

An HDI composition and a preparation method thereof were provided, wherein the effective factor E of the HDI composition was 4.70. The schematic flow chart of the preparation method is shown in FIG. 1, and specifically included the following steps.

Phosgenation process: Preheated and vaporized 1,6-hexanediamine was introduced into a phosgenation reactor at a rate of 1000 parts by mass/hour, and phosgene was simultaneously introduced at a rate of 5112 parts by mass/hour. Both of the feed streams were fed at a temperature of 310°C, passed through a reaction zone of the phosgenation reactor, and then trapped by chlorobenzene to obtain a reaction liquid. The feed pressure of 1,6-hexanediamine and phosgene was 0.25 MPa. The absolute pressure in the reaction zone was 0.09 MPa, which was slightly less than atmospheric pressure. The molar ratio of phosgene to 1, 6-hexanediamine was 6:1. In the reaction zone, the temperature was 350°C, the flow rate was 70 m/s, the average contact time period was 2 s, and the Reynolds number was 5000. Cold chlorobenzene was used for trapping at the outlet of the reactor to obtain the reaction liquid, with a temperature of 140°C after trapping. As such, 1,6-hexanediamine was reacted with phosgene to produce HDI, and a reaction product containing HDI was obtained.

Removal process: The reaction product obtained in the phosgenation process was continuously introduced into a phosgene removal tower and a solvent removal tower to perform a phosgene removal treatment and a solvent removal treatment, respectively, so as to obtain 1440 parts by mass of crude HDI.

Separation process: The crude product obtained in the removal process was continuously introduced into a short-path evaporator to obtain 1418.7 parts by mass of an intermediate fraction from which a heavy fraction was removed, and 21.3 parts by mass of a primary heavy fraction.

Heavy fraction recovering process: The primary heavy fraction was continuously introduced into a secondary short-path evaporator to obtain 14.3 parts by mass of a heavy fraction recovered material and 7.0 parts by mass of a residual heavy fraction, wherein the heavy fraction recovered material can be obtained through multiple cycles of the short-path evaporator. Next, the intermediate fraction at a rate of 1418.7 parts by mass/hour and the heavy fraction recovered material at a rate of 14.3 parts by mass/hour were mixed to obtain a mixture, wherein a mass content percentage of the heavy fraction recovered material in the mixture was 1%.

Refining process: The mixture was continuously introduced at a rate of 1433 parts by mass/hour into a rectification tower, then a light fraction was removed from the top of the tower, and an HDI composition was collected from the tower to obtain a target product. The rectification tower was filled with a packing corresponding to a theoretical plate number of 25.

The rectification conditions in the rectification tower were as follows:

Bottom Temperature: 120°C to130°C.
Top temperature: 80°C to 100°C.
Top pressure: 10-50 PaA.
Residence time: 2-3 h.
Top reflux ratio: 10.
Output of the refining process: 1329 parts by mass/hour.

Thus, the HDI composition was obtained, wherein the mass content of HDI is greater than 99%, the mass content of chlorine (A value) is 22.2 ppm, the mass content of chlorinated isocyanate CHI (B value) is 10 ppm, and the effective factor E is 4.70.

Examples 2 to 5 and Comparative Examples 1 to 2

HDI compositions and preparation methods thereof were provided, wherein the effective factors E of these HDI compositions are shown in Table 1, respectively. The preparation methods were similar to that in Example 1, except that some process parameters were different, which were specifically shown in Table 1. The processes/parameters not shown in Table 1 were completely the same as those of Example 1. In Table 1, the "phosgene molar proportion" represents the mole amount of phosgene in the phosgenation process based on 1 mol of 1,6-hexanediamine. The "heavy fraction recovered material proportion" represents the mass content percentage of the heavy fraction (recovered material) in the mixture in the heavy fraction recovering process.

Table 1

| | | Example 1 | Example 2 | Example 3 | Example 4 | Example 5 | Comparative Example 1 | Comparative Example 2 |
|---|---|---|---|---|---|---|---|---|
| Phosgenation process | Phosgene introducing rate (parts by mass /h) | 5112 | 5964 | 6816 | 7668 | 8519 | 5112 | 8519 |
| | Phosgene molar proportion | 6 | 7 | 8 | 9 | 10 | 6 | 10 |
| | Reaction temperature (°C) | 350 | 375 | 400 | 425 | 450 | 350 | 450 |
| Heavy fraction recovering process | Mixture (parts by mass/h) | 1433.0 | 1418.6 | 1418.6 | 1404.1 | 1404.1 | 1433.0 | 1404.1 |
| | Heavy fraction recovered material (parts by mass/h) | 14.3 | 31.2 | 63.8 | 106.7 | 140.4 | 7.2 | 168.5 |
| | Heavy fraction recovered material proportion (%) | 1.0 | 2.2 | 4.5 | 7.6 | 10 | 0.5 | 12 |
| Refining process | output (parts by mass/h) | 1329 | 1355 | 1372 | 1387 | 1411 | 1323 | 1418 |
| | Reflux ratio | 10 | 8 | 8 | 6 | 5.5 | 5.5 | 10 |
| HDI composition | HDI (%) | >99 | >99 | >99 | >99 | >99 | >99 | >99 |
| | Chlorine (ppm) | 22.2 | 87 | 225 | 536 | 853 | 19 | 893 |
| | CHI (ppm) | 10 | 105 | 510 | 1483 | 2970 | 20 | 3150 |
| | Effective factor E | 4.70 | 4.19 | 3.95 | 3.77 | 3.70 | 4.82 | 3.65 |

Examples 6 to 10 and Comparative Examples 3 to 4

PDI compositions and preparation methods thereof were provided, wherein the effective factors E of these PDI

compositions are shown in Table 2, respectively. The preparation methods were similar to that in Example 1, except that some process parameters were different, which are specifically shown in Table 2. The processes/parameters not shown in Table 2 were completely the same as those of Example 1. In Table 2, the "phosgene molar proportion" represents the mole amount of phosgene in the phosgenation process based on 1 mol of 1,5-pentanediamine. The "heavy fraction recovered material proportion" represents the mass content percentage of the heavy fraction (recovered material) in the mixture in the heavy fraction recovering process.

Table 2

| | | Exampl e6 | Exampl e 7 | Exampl e8 | Exampl e9 | Exampl e 10 | Comparati ve Example 3 | Comparati ve Example 4 |
|---|---|---|---|---|---|---|---|---|
| Phosgenati on process | Phosgene introducin g rate (parts by mass /h) | 5812 | 6781 | 7750 | 8719 | 9686 | 5812 | 9686 |
| | Phosgene molar proportio n | 6 | 7 | 8 | 9 | 10 | 6 | 10 |
| | Reaction temperatu re (°C) | 350 | 375 | 400 | 425 | 450 | 350 | 450 |
| Heavy frac-tion reco-vering pro-cess | Mixture (parts by mass/h) | 1490.3 | 1475.3 | 1475.3 | 1460.3 | 1460.3 | 1490.3 | 1460.3 |
| | Heavy fraction recovered material (parts by mass/h) | 14.9 | 32.5 | 66.4 | 111.0 | 146.0 | 7.5 | 175.2 |
| | Heavy fraction recovered material proportio n (%) | 1 | 2.2 | 4.5 | 7.6 | 10 | 0.5 | 12 |
| Refining process | output (parts by mass/h) | 1385 | 1413 | 1430 | 1446 | 1471 | 1379 | 1478 |
| | Reflux ratio | 10 | 8 | 8 | 6 | 5.5 | 5.5 | 10 |
| PDI compositio n | PDI (%) | >99 | >99 | >99 | >99 | >99 | >99 | >99 |
| | Chlorine (ppm) | 25.5 | 82 | 207 | 436 | 768 | 8.8 | 932 |
| | CPI (ppm) | 23 | 125 | 480 | 1281 | 2570 | 16 | 2960 |
| | Effective factor E | 4.70 | 4.28 | 4.04 | 3.89 | 3.82 | 5.30 | 3.68 |

Examples 11 to 15 and Comparative Examples 5 to 6

HMDI compositions and preparation methods thereof were provided, wherein the effective factors E of these HMDI compositions are shown in Table 3, respectively. The preparation methods were similar to that in Example 1, except that some process parameters were different, which are specifically shown in Table 3. The processes/parameters not shown in Table 3 are completely the same as those of Example 1. In Table 3, the "phosgene molar proportion" represents the mole amount of phosgene in the phosgenation process based on 1 mol of 4,4'-diaminodicyclohexylmethane. The "heavy fraction recovered material proportion" represents the mass content percentage of the heavy fraction (recovered material) in the mixture in the heavy fraction recovering process.

Table 3

| | | Exampl e 11 | Exampl e 12 | Exampl e13 | Exampl e 14 | Exampl e 15 | Comparati ve Example 5 | Comparati ve Example 6 |
|---|---|---|---|---|---|---|---|---|
| Phosgenati on process | Phosgene introducin g rate (parts by mass/h) | 2823 | 3294 | 3764 | 4235 | 4705 | 2823 | 4705 |
| | Phosgene molar proportio n | 6 | 7 | 8 | 9 | 10 | 6 | 10 |
| | Reaction temperatu re (°C) | 350 | 375 | 400 | 425 | 450 | 350 | 450 |
| Heavy frac-tion reco-vering pro-cess | Mixture (parts by mass/h) | 1234.5 | 1222.1 | 1222.1 | 1209.6 | 1209.6 | 1234.5 | 1209.6 |
| | Heavy fraction recovered material (parts by mass/h) | 12.3 | 26.9 | *55.0* | 91.9 | 121.0 | 6.2 | 145.2 |
| | Heavy fraction recovered material proportio n (%) | 1 | 2.2 | 4.5 | 7.6 | 10 | 0.5 | 12 |
| Refining process | output (parts by mass/h) | 1145 | 1167 | 1182 | 1195 | 1216 | 1140 | 1222 |
| | Reflux ratio | 10 | 8 | 8 | 6 | 5.5 | 5.5 | 10 |
| HMDI compositio n | HMDI (%) | >99 | >99 | >99 | >99 | >99 | >99 | >99 |
| | Chlorine (ppm) | 21.4 | 56 | 112 | 185 | 277.6 | 19 | 297 |
| | CHMI (ppm) | 10 | 35 | 162 | 245 | 560 | 10 | 560 |
| | Effective factor E | 4.70 | 4.29 | 4.05 | 3.82 | 3.70 | 4.75 | 3.66 |

Examples 16 to 20 and Comparative Examples 7 to 8

IPDI compositions and preparation methods thereof were provided, wherein the effective factors E of these IPDI compositions are shown in Table 4, respectively. The preparation methods were similar to that in Example 1, except that some process parameters are different, which are specifically shown in Table 3. The processes/parameters not shown in Table 4 are completely the same as those of Example 1. In Table 4, the "phosgene molar proportion" represents the mole amount of phosgene in the phosgenation process based on 1 mol of isophorone diamine. The "heavy fraction recovered material proportion" represents the mass content percentage of the heavy fraction (recovered material) in the mixture in the heavy fraction recovering process.

Table 4

| | | Example 16 | Example 17 | Example 18 | Example 19 | Example 20 | Comparative Example 7 | Comparative Example 8 |
|---|---|---|---|---|---|---|---|---|
| Phosgenation process | Phosgene introducing rate (parts by mass/h) | 3488 | 4069 | 4651 | 5232 | 5813 | 3488 | 5813 |
| | Phosgene molar proportion | 6 | 7 | 8 | 9 | 10 | 6 | 10 |
| | Reaction temperature (°C) | 350 | 375 | 400 | 425 | 450 | 350 | 450 |
| Heavy fraction recovering process | Mixture (parts by mass/h) | 1292.3 | 1279.3 | 1279.3 | 1266.2 | 1266.2 | 1292.3 | 1266.2 |
| | Heavy fraction recovered material (parts by mass/h) | 12.9 | 28.1 | 57.6 | 96.2 | 126.6 | 6.5 | 151.9 |
| | Heavy fraction recovered material proportion (%) | 1 | 2.2 | 4.5 | 7.6 | 10 | 0.5 | 12 |
| Refining process | output (parts by mass/h) | 1198 | 1222 | 1237 | 1251 | 1272 | 1193 | 1279 |
| | Reflux ratio | 10 | 8 | 8 | 6 | 5.5 | 5.5 | 10 |
| IPDI composition | IPDI (%) | >99 | >99 | >99 | >99 | >99 | >99 | >99 |
| | Chlorine (ppm) | 22.5 | 55 | 104 | 206 | 307.5 | 17 | 357 |
| | CIPI (ppm) | 15 | 37 | 152 | 345 | 653 | 15 | 653 |
| | Effective factor E | 4.70 | 4.31 | 4.1 | 3.83 | 3.70 | 4.84 | 3.6 |

Examples 21 to 24 and Comparative Examples 9 to 12

As Comparative Example 9, HDI was prepared by the method described in Example 8 of related art CN101962348A. The HDI product in Comparative Example 9 was prepared by pyrolysis, and did not contain chlorine, that is, no effective factor was obtained. The heavy fraction recovered material in Example 1 was added to the HDI product at a ratio of 4% (i.e., the mass content percentage of the heavy fraction in the resulting mixture was 4%) to obtain Example 21.

Similarly, as Comparative Example 10, PDI was prepared by the method described in Example 1 of related art CN114105825A. The heavy fraction recovered material in Example 6 was added to the PDI product at a ratio of 4% (i.e., the mass content percentage of heavy fraction in the resulting mixture was 4%) to obtain Example 22.

As Comparative Example 11, HMDI was prepared by the method described in Example 4 of related art CN101234998A. The heavy fraction recovered material in Example 11 was added to the HMDI product at a ratio of 4% (i.e., the mass content percentage of the heavy fraction in the resulting mixture was 4%) to obtain Example 23.

As Comparative Example 12, IPDI was prepared by the method described in Example 1 of related art CN114507161A. The heavy fraction recovered material in Example 16 was added to the IPDI product at a ratio of 4% (i.e., the mass content percentage of the heavy fraction in the resulting mixture was 4%) to obtain Example 24.

Application Example

A two-component polyurethane composition, in particular a two-component polyurethane coating material (a coating material), included an agent A and an agent B.

Specifically, this application example provided two agents A, denoted as agent A-1 and agent A-2, which were respectively combined with the agent B to form the two-component polyurethane coating material. The formulation was as follows:

(1) Preparation of agent A-1: 413.7 parts by mass of the HDI composition (37.7 parts by mass of the PDI composition, 39.9 parts by mass of the HMDI composition, or 39.9 parts by mass of the IPDI composition) and 36.7 parts by mass of trimethylolpropane were mixed, and reacted at 70°C for 6 h under a nitrogen gas atmosphere. The reaction solution obtained after the reaction was distilled with a thin film distillation device to remove unreacted isocyanate, thereby obtaining a modified isocyanate composition, which contains carbamate group obtained by reacting isocyanate with trimethylolpropane. The above-mentioned isocyanate compositions were the isocyanate compositions provided in Examples 1 to 24 and Comparative Examples 1 to 12, respectively.
In a form of a solid component being 75 wt.%, ethyl acetate was added to the modified isocyanate composition to produce a polyisocyanate component (i.e., agent A-1), wherein the content of NCO group in the polyisocyanate component is 11.6 wt.%.
(2) Preparation of agent A-2: 2 parts by mass of 1,3-butanediol were added to 100 parts by mass of the HDI composition (37.7 parts by mass of the PDI composition, 39.9 parts by mass of the HMDI composition, or 39.9 parts by mass of the IPDI composition), raised to a temperature of 75°C under a nitrogen gas atmosphere, and subjected to a carbamate esterification for 2 hours, wherein an equivalent ratio (NCO/OH) of the isocyanate group in the isocyanate composition to the hydroxyl group in 1,3-butanediol is 24. Then, at the same temperature, as the isocyanurate esterification catalyst, 0.1 phr (converted to a solid component of 0.037 phr) of a tetrabutylammonium hydroxide solution (a solution having 37% methanol) was combined to perform the isocyanurate esterification reaction for 4 hours. The obtained reaction solution passed through a thin film distillation device at a temperature of 150°C and a vacuum degree of 50 Pa to remove unreacted isocyanate, with a distillation yield of 60 wt.%, thereby obtaining a modified isocyanate composition including isocyanurate groups of an isocyanate trimer. The above-mentioned isocyanate compositions were the isocyanate compositions provided in Examples 1 to 24 and Comparative Examples 1 to 12, respectively.
In a form of a solid content being 75 wt.%, ethyl acetate was added to the modified isocyanate composition to produce a polyisocyanate component (i.e., agent A-2).
(3) Preparation of agent B: In parts by mass, 40 parts of fluoropolyol (ZEFFLE GK-570, manufactured by DAIKIN INDUSTRIES LTD., hydroxyl value of solid content: 64 mg KOH/g, solvent: butyl acetate), 52.5 parts of titanium oxide (CR93, manufactured by Ishihara Sangyo Kaisha, Ltd.,), 33.8 parts of butyl acetate, and 110 parts of glass beads with a diameter of 2 mm were stirred with a paint stirrer for 2 h, and then was filtered to remove the glass beads. Next, in a form of a solid content being 58 wt.%, butyl acetate was added to obtain the agent B, wherein the proportion of titanium oxide was 45 wt.%
(4) Preparation of two-component polyurethane coating material: The obtained agent A (agent A-1 or agent A-2) and agent B were mixed at an equivalent ratio of isocyanate group to hydroxyl group (NCO/OH) of 1.0 to obtain a mixed liquid. Butyl acetate was added to the mixed liquid at a NV value (a mass of coating component, a solid content) of 60 wt.% to obtain the two-component polyurethane coating material.

Performance Evaluation

The two-component polyurethane coating material to be tested was coated on the surface of a polyethylene terephthalate (PET) substrate and cured by heating at 120°C for 2 min. Thereafter, the PET substrate coated with the mixed solution was maintained at 60°C for 2 days, thereby forming a coating layer having a thickness of about 15 μm on the PET substrate.

The weather resistance of the coating layer, reflected by a color difference of the coating layer in the damp heat test, was detected as follows. An initial b value (b1, an initial value) of the coating layer was measured using a colorimeter (3 nh NR10QC). Then, the sample coated with the coating layer was placed in a constant temperature and humidity device (a high-iron device) and maintained for 2000 h at a temperature of 85°C and a relative humidity of 85%. The b value (b2) of the sample underwent the damp heat test for 2000 h was measured by the same method as above, and the color difference Δb of the coating layer before and after the damp heat test was calculated, where Δb=|b2-b1|. The results are shown in Tables 5 to 8.

Table 5

| | | Example 1 | Example 2 | Example 3 | Example 4 | Example 5 | Example 21 | Compa rative Example 1 | Compa rative Example 2 | Compa rative Example 9 |
|---|---|---|---|---|---|---|---|---|---|---|
| HDI composit ion | HDI (%) | >99 | >99 | >99 | >99 | >99 | >99 | >99 | >99 | >99 |
| | Chlorine (ppm) | 22.2 | 87 | 225 | 536 | 853 | 25 | 19 | 893 | - |
| | CHI (ppm) | 10 | 105 | 510 | 1483 | 2970 | 15 | 20 | 3150 | - |
| | Effective factor E | 4.70 | 4.19 | 3.95 | 3.77 | 3.70 | 4.66 | 4.82 | 3.65 | - |
| coating layer with Agent A-1 | b1 | 0.38 | 0.39 | 0.42 | 0.42 | 0.46 | 0.39 | 0.40 | 0.47 | 0.38 |
| | b2 | 1.36 | 1.39 | 1.44 | 1.47 | 1.52 | 1.37 | 1.75 | 1.86 | 1.72 |
| | Δb | 0.98 | 1 | 1.02 | 1.05 | 1.06 | 0.98 | 1.35 | 1.39 | 1.34 |
| coating layer with Agent A-2 | b1 | 0.42 | 0.42 | 0.46 | 0.46 | 0.51 | 0.43 | 0.43 | 0.52 | 0.42 |
| | b2 | 1.45 | 1.47 | 1.53 | 1.56 | 1.62 | 1.47 | 1.79 | 1.99 | 1.76 |
| | Δb | 1.03 | 1.05 | 1.07 | 1.1 | 1.11 | 1.04 | 1.36 | 1.47 | 1.34 |

Table 6

| | | Example 6 | Example 7 | Example 8 | Example 9 | Example 10 | Example 22 | Comparative Example 3 | Comparative Example 4 | Comparative Example 10 |
|---|---|---|---|---|---|---|---|---|---|---|
| PDI composition | PDI (%) | >99 | >99 | >99 | >99 | >99 | >99 | >99 | >99 | >99 |
| | Chlorine (ppm) | 25.5 | 82 | 207 | 436 | 768 | 31 | 8.8 | 932 | - |
| | CPI (ppm) | 23 | 125 | 480 | 1281 | 2570 | 35 | 16 | 2960 | - |
| | Effective factor E | 4.70 | 4.28 | 4.04 | 3.89 | 3.82 | 4.65 | 5.30 | 3.68 | - |
| coating layer with Agent A-1 | b1 | 0.39 | 0.40 | 0.43 | 0.43 | 0.45 | 0.41 | 0.40 | 0.49 | 0.40 |
| | b2 | 1.39 | 1.42 | 1.47 | 1.50 | *1.55* | 1.43 | 1.72 | 1.87 | 1.70 |
| | Δb | 1.00 | 1.02 | 1.04 | 1.07 | 1.10 | 1.02 | 1.32 | 1.38 | 1.3 |
| coating layer with Agent A-2 | b1 | 0.43 | 0.43 | 0.47 | 0.47 | 0.48 | 0.43 | 0.44 | 0.52 | 0.43 |
| | b2 | 1.48 | 1.50 | 1.56 | 1.59 | 1.65 | 1.49 | 1.83 | 1.98 | 0.74 |
| | Δb | 1.05 | 1.07 | 1.09 | 1.12 | 1.17 | 1.06 | 1.39 | 1.46 | 1.31 |

Table 7

| | | Example 11 | Example 12 | Example 13 | Example 14 | Example 15 | Example 23 | Comparative Example 5 | Comparative Example 6 | Comparative Example 11 |
|---|---|---|---|---|---|---|---|---|---|---|
| HMDI composition | HMDI(%) | >99 | >99 | >99 | >99 | >99 | >99 | >99 | >99 | >99 |
| | Chlorine (ppm) | 21.4 | 56 | 112 | 185 | 277.6 | 25 | 19 | 297 | - |
| | CHMI (ppm) | 10 | 35 | 162 | 245 | 560 | 26 | 10 | 560 | - |
| | Effective factor E | 4.70 | 4.29 | 4.05 | 3.82 | 3.70 | 4.67 | 4.75 | 3.66 | - |
| coating layer with Agent A-1 | b1 | 0.41 | 0.42 | 0.43 | 0.45 | 0.47 | 0.42 | 0.42 | 0.49 | 0.41 |
| | b2 | 1.46 | 1.49 | 1.53 | 1.57 | 1.62 | 1.48 | 1.81 | 1.96 | 1.76 |
| | Δb | 1.05 | 1.07 | 1.10 | 1.12 | 1.15 | 1.06 | 1.39 | 1.47 | 1.35 |
| coating layer with Agent A-2 | b1 | 0.44 | 0.44 | 0.47 | 0.49 | 0.51 | 0.44 | 0.45 | 0.53 | 0.44 |
| | b2 | 1.52 | 1.54 | 1.61 | 1.65 | 1.70 | 1.53 | 1.87 | 2.04 | 1.80 |
| | Δb | 1.08 | 1.10 | 1.14 | 1.16 | 1.19 | 1.09 | 1.42 | 1.51 | 1.36 |

Table 8

| | | Example 16 | Example 17 | Example 18 | Example 19 | Example 20 | Example 24 | Comparative Example 7 | Comparative Example 8 | Comparative Example 12 |
|---|---|---|---|---|---|---|---|---|---|---|
| IPDI composition | IPDI (%) | >99 | >99 | >99 | >99 | >99 | >99 | >99 | >99 | >99 |
| | Chlorine (ppm) | 22.5 | 55 | 104 | 206 | 307.5 | 28 | 17 | 357 | -- |
| | CIPI (ppm) | 15 | 37 | 152 | 345 | 653 | 25 | 15 | 653 | -- |
| | Effective factor E | 4.70 | 4.31 | 4.1 | 3.83 | 3.70 | 4.62 | 4.84 | 3.6 | -- |
| coating layer with Agent A-1 | b1 | 0.40 | 0.41 | 0.42 | 0.44 | 0.46 | 0.41 | 0.41 | 0.48 | 0.40 |
| | b2 | 1.44 | 1.46 | 1.51 | *1.55* | 1.60 | 1.45 | 1.80 | 1.94 | 1.75 |
| | Δb | 1.04 | 1.05 | 1.09 | 1.11 | 1.14 | 1.04 | 1.39 | 1.46 | 1.35 |
| coating layer with Agent A-2 | b1 | 0.43 | 0.43 | 0.46 | 0.48 | 0.50 | 0.44 | 0.51 | 0.52 | 0.43 |
| | b2 | 1.51 | 1.54 | 1.59 | 1.63 | 1.69 | 1.52 | 1.92 | 2.02 | 1.80 |
| | Δb | 1.08 | 1.11 | 1.13 | 1.15 | 1.19 | 1.08 | 1.41 | 1.50 | 1.37 |

Form the above performance test data, it can be seen that by controlling the effective factor of the isocyanate composition in the range from 3.70 to 4.70, the prepared two-component polyurethane coating material exhibits excellent discoloration resistance, and good color stability at high temperature and high humidity. The color difference Δb of the coating layer after the damp heat durability test (2000 h) is less than 1.2, as low as 0.98 to 1.19.

The applicant states that the isocyanate composition, its preparation method and use of the present application are described with reference to the above examples, but the present application is not limited to the above-mentioned process steps, that is, it does not mean that the present application must rely on the above-mentioned process steps to be implemented. It should be understood by those skilled in the art that any improvement to the present application, equivalent substitution of raw materials selected in the present application, addition of auxiliary ingredients, selection of specific methods, etc., shall fall within the scope of protection and disclosure of the present application.

## Claims

**1.** An isocyanate composition, wherein an effective factor of the isocyanate composition is in a range from 3.70 to 4.70;

the effective factor is calculated according to Formula I:

$$E = -\lg\left(A - \frac{B \times M_{Cl}}{M_B}\right)$$ Formula I;

where E is the effective factor;
A is a mass content of chlorine in the isocyanate composition;
B is a mass content of chlorinated isocyanate in the isocyanate composition;
$M_{C1}$ is a relative atomic mass of chlorine; and
$M_B$ is a relative molecular mass of the chlorinated isocyanate.

**2.** The isocyanate composition according to claim 1, wherein the isocyanate is a diisocyanate.

**3.** The isocyanate composition according to claim 2, wherein the diisocyanate comprises any one or a combination of at

least two of pentamethylene diisocyanate, hexamethylene diisocyanate, dicyclohexylmethane diisocyanate, or isophorone diisocyanate.

4. The isocyanate composition according to any one of claims 1 to 3, wherein a mass content percentage of the isocyanate in the isocyanate composition is greater than or equal to 97%.

5. The isocyanate composition according to any one of claims 1 to 4, wherein a substance corresponding to the effective factor comprises any one or a combination of at least two of following compounds:

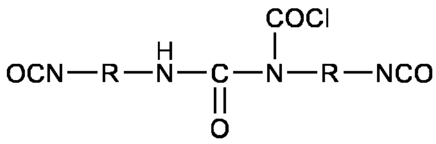 , 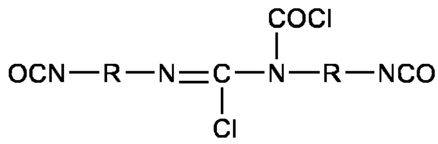 ,

or

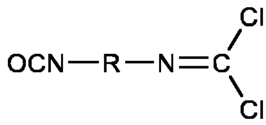 ,

where R is a divalent group obtained after removing NCO group from the isocyanate;
preferably, R is selected from any one or a combination of at least two of

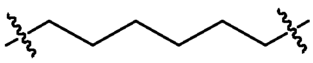 , 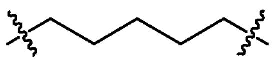 , 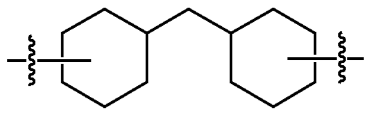 ,

or

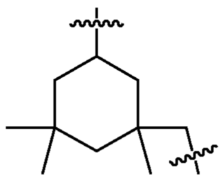 ,

where a wavy line represents an attachment site of the group.

6. The isocyanate composition according to any one of claims 1 to 5, wherein the chlorinated isocyanate is a compound obtained by replacing one NCO group in the isocyanate with chlorine; preferably, the chlorinated isocyanate comprises any one or a combination of at least two of

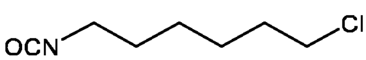 , 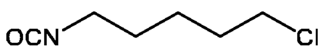 , 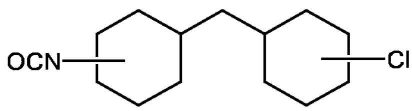 ,

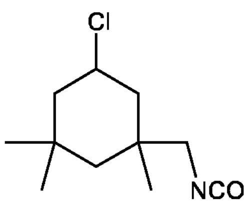 , or 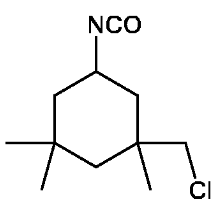 .

7. The isocyanate composition according to any one of claims 1 to 6, wherein the A is determined by X-ray fluorescence spectroscopy;
preferably, the B is determined by chromatography-mass spectrometry, further preferably by gas chromatography-mass spectrometry.

8. A method for preparing the isocyanate composition according to any one of claims 1 to 7, comprising reacting an amine compound with phosgene to obtain the isocyanate composition.

9. The method according to claim 8, comprising the steps of:

(1) reacting the amine compound with phosgene to obtain a reaction product;
(2) subjecting the reaction product obtained in step (1) to a removal treatment to obtain a crude product, wherein the removal treatment comprises a phosgene removal treatment and/or a solvent removal treatment; and
(3) sequentially separating and refining the crude product obtained in step (2) to obtain the isocyanate composition.

10. The method according to claim 9, wherein in step (3), a heavy fraction and an intermediate fraction are obtained after the separation, a mixture of the intermediate fraction and the heavy fraction is refined to obtain the isocyanate composition, and a mass content percentage of the heavy fraction in the mixture is in a range from 1% to 10%.

11. The method according to claim 10, wherein the refining process is rectification.

12. A modified isocyanate composition, wherein the modified isocyanate composition is obtained by modifying the isocyanate composition of any one of claims 1 to 7;
the modified isocyanate composition comprises any one or a combination of at least two of groups (a) to (i): (a) isocyanurate group, (b) uretdione group, (c) biuret group, (d) carbamate group, (e) ureido group, (f) iminooxadiazinedione group, (g) allophanate group, (h) uretonimine group, or (i) carbodiimide group.

13. An isocyanate-based polymer, wherein the polymer is prepared by reacting an isocyanate-based substance with an active hydrogen group-containing substance, and the isocyanate-based substance comprises at least one of the isocyanate composition of any one of claims 1 to 7 or the modified isocyanate composition of claim 12.

14. A two-component polyurethane composition, wherein the two-component polyurethane composition comprises an agent A and an agent B;

the agent A comprises the isocyanate composition of any one of claims 1 to 7 and/or the modified isocyanate composition of claim 12; and
the agent B comprises an active hydrogen group-containing substance.

15. The two-component polyurethane composition according to claim 14, wherein the modified isocyanate composition comprises (a) isocyanurate group and/or (d) carbamate group;
preferably, the agent B comprises a polyol.

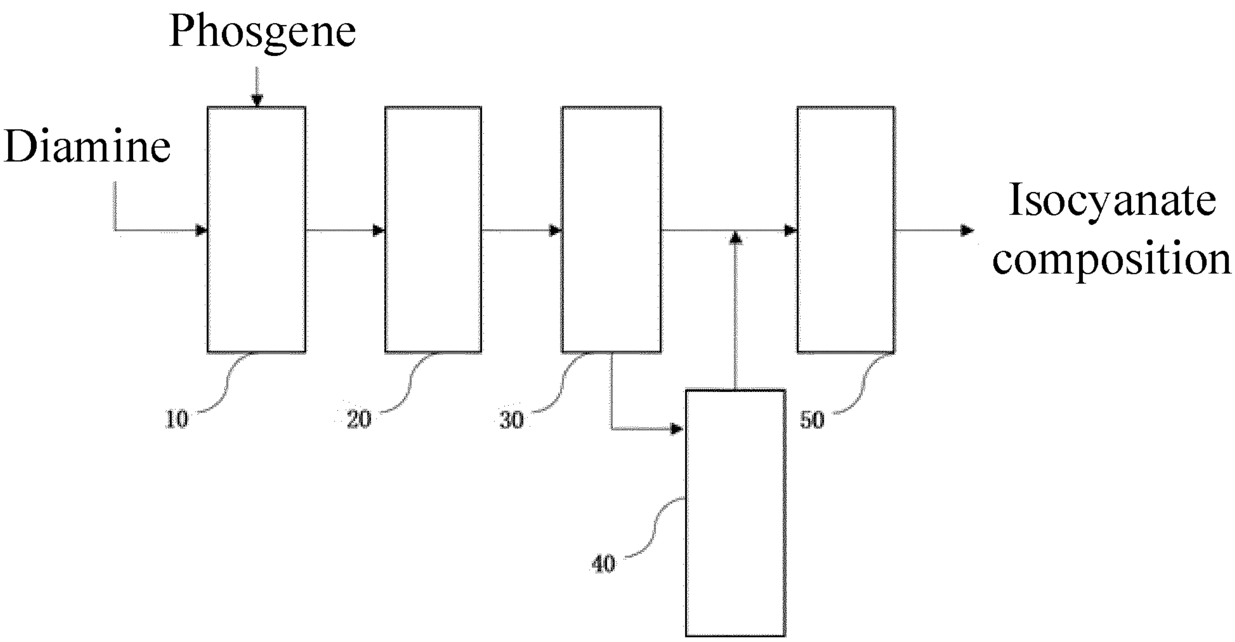


FIG. 1

**INTERNATIONAL SEARCH REPORT**

International application No.
**PCT/CN2022/132423**

**A. CLASSIFICATION OF SUBJECT MATTER**

C08G18/71(2006.01)i; C08G18/75(2006.01)i; C08G18/73(2006.01)i; C08G18/77(2006.01)i; C08G18/78(2006.01)i; C08G18/79(2006.01)i; C07C263/04(2006.01)i; C07C263/10(2006.01)i; C07C265/14(2006.01)i; C09D175/04(2006.01)i

According to International Patent Classification (IPC) or to both national classification and IPC

**B. FIELDS SEARCHED**

Minimum documentation searched (classification system followed by classification symbols)

IPC: C08G C07C C09D

Documentation searched other than minimum documentation to the extent that such documents are included in the fields searched

Electronic data base consulted during the international search (name of data base and, where practicable, search terms used)

CNTXT; ENTXTC; ENTXT; WPABS; WPABSC; DWPI; VEN; CJFD; CNKI, 万方, WANFANG, 读秀, DUXIU, 百度, BAIDU, 百度学术, BAIDU SCHOLAR, web of science: 万华化学, 朱付林, 尚永华, 李文滨, 李建峰, 俞涛, 王京旭, 何伟, 韩金平, 俞勇, 黎源, +isocyanat+, colo?r 1w stability, discolo?ration resistance, delta b, chlorine, 异氰酸酯, 因子, 有效因子, 氯, 耐变色, 颜色, 稳定, 重组分, 含量, 比例, 回收.

**C. DOCUMENTS CONSIDERED TO BE RELEVANT**

| Category* | Citation of document, with indication, where appropriate, of the relevant passages | Relevant to claim No. |
|---|---|---|
| A | JP 2010265364 A (MITSUI CHEMICALS INC.) 25 November 2010 (2010-11-25)<br>description, paragraphs 14-15 | 1-15 |
| A | CN 109761855 A (WANHUA CHEMICAL GROUP CO., LTD.) 17 May 2019 (2019-05-17)<br>description, paragraphs 4-10 | 1-15 |
| A | CN 101234998 A (EVONIK DEGUSSA GMBH) 06 August 2008 (2008-08-06)<br>description, pages 1-2 | 1-15 |
| A | CN 101440046 A (BAYER MATERIALSCIENCE AG et al.) 27 May 2009 (2009-05-27)<br>description, pages 1-2 | 1-15 |
| A | CN 101962348 A (LANZHOU INSTITUTE OF CHEMICAL PHYSICS, CHINESE ACADEMY OF SCIENCES) 02 February 2011 (2011-02-02)<br>abstract | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **2023-07-31** | **02 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2022/132423**

**C. DOCUMENTS CONSIDERED TO BE RELEVANT**

| Category* | Citation of document, with indication, where appropriate, of the relevant passages | Relevant to claim No. |
|---|---|---|
| A | CN 103319372 A (WANHUA CHEMICAL GROUP CO., LTD. et al.) 25 September 2013 (2013-09-25) description, paragraphs 9-15 | 1-15 |
| A | CN 113651719 A (NINGXIA RUITAI TECHNOLOGY CO., LTD. et al.) 16 November 2021 (2021-11-16) abstract | 1-15 |
| A | CN 114105825 A (INSTITUTE OF PROCESS ENGINEERING, CHINESE ACADEMY OF SCIENCES) 01 March 2022 (2022-03-01) description, paragraphs 2-10 | 1-15 |
| A | CN 114507161 A (INSTITUTE OF PROCESS ENGINEERING, CHINESE ACADEMY OF SCIENCES) 17 May 2022 (2022-05-17) description, paragraphs 2-4 | 1-15 |
| A | CN 115073707 A (WANHUA CHEMICAL GROUP CO., LTD.) 20 September 2022 (2022-09-20) description, paragraphs 1-51 | 1-15 |
| A | CN 1183791 A (ASAHI CHEMICAL INDUSTRY CO., LTD.) 03 June 1998 (1998-06-03) abstract | 1-15 |
| A | CN 1356980 A (BASF AG) 03 July 2002 (2002-07-03) description, pages 1-4 | 1-15 |
| A | KR 20050108563 A (KUMHO MITSUI CHEMICALS INC.) 17 November 2005 (2005-11-17) claim 1, and description, pages 1-2 | 1-15 |
| A | WO 2022128925 A1 (BASF SE) 23 June 2022 (2022-06-23) description, pages 1-3 | 1-15 |

Form PCT/ISA/210 (second sheet) (July 2022)

**INTERNATIONAL SEARCH REPORT**
**Information on patent family members**

International application No.
**PCT/CN2022/132423**

| Patent document cited in search report | Publication date (day/month/year) | Patent family member(s) | Publication date (day/month/year) |
|---|---|---|---|
| JP 2010265364 A | 25 November 2010 | JP 5270446 B2 | 21 August 2013 |
| CN 109761855 A | 17 May 2019 | WO 2020124622 A1 | 25 June 2020 |
| | | JP 2022504766 A | 13 January 2022 |
| | | JP 7194822 B2 | 22 December 2022 |
| | | KR 20210068086 A | 08 June 2021 |
| | | EP 3901133 A1 | 27 October 2021 |
| | | EP 3901133 A4 | 05 October 2022 |
| | | US 2022002235 A1 | 06 January 2022 |
| | | CN 109761855 B | 28 July 2020 |
| CN 101234998 A | 06 August 2008 | US 2010036154 A1 | 11 February 2010 |
| | | US 8816125 B2 | 26 August 2014 |
| | | ES 2541782 T3 | 24 July 2015 |
| | | WO 2008077672 A1 | 03 July 2008 |
| | | EP 2091911 A1 | 26 August 2009 |
| | | EP 2091911 B1 | 29 April 2015 |
| | | DE 102006061475 A1 | 26 June 2008 |
| CN 101440046 A | 27 May 2009 | JP 2015110640 A | 18 June 2015 |
| | | KR 20090050002 A | 19 May 2009 |
| | | KR 101602495 B1 | 10 March 2016 |
| | | EP 2060560 A1 | 20 May 2009 |
| | | EP 2060560 B1 | 13 April 2016 |
| | | ES 2582173 T3 | 09 September 2016 |
| | | HUE 027861 T2 | 28 November 2016 |
| | | JP 2009149614 A | 09 July 2009 |
| | | RU 2008144792 A | 20 May 2010 |
| | | RU 2487865 C2 | 20 July 2013 |
| | | US 2009149671 A1 | 11 June 2009 |
| | | US 9382198 B2 | 05 July 2016 |
| | | CN 101440046 B | 18 September 2013 |
| CN 101962348 A | 02 February 2011 | US 2011021810 A1 | 27 January 2011 |
| | | US 8809574 B2 | 19 August 2014 |
| CN 103319372 A | 25 September 2013 | CN 103319372 B | 05 November 2014 |
| CN 113651719 A | 16 November 2021 | None | |
| CN 114105825 A | 01 March 2022 | WO 2022041502 A1 | 03 March 2022 |
| CN 114507161 A | 17 May 2022 | None | |
| CN 115073707 A | 20 September 2022 | WO 2022188825 A1 | 15 September 2022 |
| CN 1183791 A | 03 June 1998 | AU 6242696 A | 05 February 1997 |
| | | TW 438869 B | 07 June 2001 |
| | | DE 69625203 D1 | 16 January 2003 |
| | | DE 69625203 T2 | 28 May 2003 |
| | | AT 229044 T | 15 December 2002 |
| | | WO 9702303 A1 | 23 January 1997 |
| | | EP 0835889 A1 | 15 April 1998 |
| | | EP 0835889 A4 | 07 October 1998 |
| | | EP 0835889 B1 | 04 December 2002 |
| | | KR 19990007853 A | 25 January 1999 |
| | | KR 100242360 B1 | 01 February 2000 |
| | | US 5852111 A | 22 December 1998 |
| | | CN 1085682 C | 29 May 2002 |

Form PCT/ISA/210 (patent family annex) (July 2022)

**INTERNATIONAL SEARCH REPORT**
**Information on patent family members**

International application No.
**PCT/CN2022/132423**

| Patent document cited in search report | Publication date (day/month/year) | Patent family member(s) | Publication date (day/month/year) |
|---|---|---|---|
| CN 1356980 A | 03 July 2002 | ES 2206268 T3 | 16 May 2004 |
| | | EP 1187808 A1 | 20 March 2002 |
| | | EP 1187808 B1 | 20 August 2003 |
| | | JP 2003503383 A | 28 January 2003 |
| | | JP 4722352 B2 | 13 July 2011 |
| | | AT 247635 T | 15 September 2003 |
| | | HU 0201848 A2 | 28 September 2002 |
| | | HU 222987 B1 | 28 January 2004 |
| | | PT 1187808 E | 31 December 2003 |
| | | US 6900348 B1 | 31 May 2005 |
| | | DE 19928741 A1 | 28 December 2000 |
| | | WO 0100569 A1 | 04 January 2001 |
| | | KR 20020010726 A | 04 February 2002 |
| | | KR 100686302 B1 | 23 February 2007 |
| | | DE 50003380 D1 | 25 September 2003 |
| | | CN 1182108 C | 03 July 2002 |
| KR 20050108563 A | 17 November 2005 | KR 100587428 B1 | 09 June 2006 |
| WO 2022128925 A1 | 23 June 2022 | None | |

Form PCT/ISA/210 (patent family annex) (July 2022)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- CN 101440046 A
- CN 103319372 A
- CN 1356980 A
- CN 109761855 A
- CN 101962348 A
- CN 114105825 A
- CN 101234998 A
- CN 114507161 A